# EUROPEAN PATENT SPECIFICATION

(11) **EP 3 237 021 B1**
(45) Date of publication and mention of the grant of the patent: **23.12.2020**
(21) Application number: 15813879.2
(22) Date of filing: 22.12.2015
(51) Int. Cl.: A61L 2/18, A61F 13/40, B08B 1/00, C11D 17/04

(54) **SANITISING DEVICE**
ENTKEIMUNGSVORRICHTUNG
DISPOSITIF DE DÉSINFECTION

(30) Priority: 22.12.2014 GB 201422924
(43) Date of publication of application: 01.11.2017
(73) Proprietor: Flexible Medical Packaging Ltd., Loudwater, Buckinghamshire HP10 9QY (GB)
(72) Inventor: BYTHEWAY, David, Loudwater Buckinghamshire HP10 9QY (GB); GOODALL, Chris, Loudwater Buckinghamshire HP10 9QY (GB); FRYERS, David, Loudwater Buckinghamshire HP10 9QY (GB)
(74) Representative: Lock, Graham James
(86) International application number: PCT/EP2015/081058
(87) International publication number: WO 2016/102606

(56) References cited:
- EP-A2- 1 201 562
- EP-A2- 1 404 174
- US-A- 5 709 866
- US-A- 6 001 187
- US-A- 6 068 820
- US-A1- 2004 062 680
- US-A1- 2012 066 850
- US-B1- 6 945 402

## Description

The present invention relates to a device which comprises an enclosure having therein at least one applicator and at least one vessel having liquid therein. In use, the liquid is released from the vessel onto the applicator by bursting the vessel prior to opening the enclosure. Thereby, the applicator is wetted with the liquid for use prior to its removal from the enclosure. The invention is particularly useful in sanitising hard surfaces, medical supplies or equipment or for use as an antimicrobial, for example in veterinary or human wound care, but it is not limited to these uses.

### Background of the Invention

US 6068820 relates to a fluid/solution wiping system. The document discloses that a proper quantity of a desired cleaning/disinfecting liquid sufficient to wet a desired wiping material is introduced into a small plastic pouch and sealed. The wiping material and the sealed pouch are placed in a plastic bag and the bag is then sealed closed. The bag is preferably sealed over the top of the plastic pouch as well, fixing the pouch in place in the bag. The sealed bag is transported to the intended cleaning location. Thereat, the bag and pouch therein are folded over, and the pouch ruptured to release the liquid, thereby wetting the wiping material. The outer bag is then opened to provide access to the wetted wiping material.

For a number of years industry has been seeking an impregnated single use wipe for the cleansing of hospital instruments and hard surfaces. Known wipes for this purpose are generally impregnated with a disinfectant solution.

One or two part sanitising solutions are known. One part sanitising solutions generally comprise a reagent which is applied directly to a surface and then wiped over the surface with a cloth, tissue or wipe. Alternatively, the reagent can be applied to a cloth, tissue or wipe before wiping it over the surface. One such sanitising solution is hypochlorous acid which is a weak acid with the chemical formula HClO. It forms together with hydrochloric acid when chlorine dissolves in water and is often used in the sanitisation of swimming pools. It provides the advantage of being a relatively strong sanitising solution, but it suffers from the disadvantage that it is a relatively unstable. In this regard, it has been found that it suffers from the problem that it reacts and decays on coming into contact with a bacterial load to gradually become inactive.

Two part sanitising solutions are generally used in applications where the active sanitising agent would break down over time. Therefore, the sanitising solution is prepared commonly *in situ* shorty before it is to be used. One sanitising agent known for this purpose is chlorine dioxide, which can be formed by bringing together mixtures of reagents including chlorite and acid; chlorate, peroxide and acid; or chlorite, hypochlorite, and a buffer. Chlorine dioxide is used because the two pack chemistry offers opportunities where the chlorine dioxide has excellent kill rate for spores, viruses and bacteria. In this regard, it has relatively strong sanitising and bactericidal properties.

However, it is not always convenient to mix up separate batches of solutions for use in sanitising equipment. In addition, there are safety issues involved in mixing the necessary reagents. Therefore, it is common to use wipes impregnated with solutions containing alcohol, detergent or soapy water. However, these solutions are generally not as effective in sanitising or decontaminating surfaces.

In light of these problems, various proposals have been made, but there remains a need for an improved solution which addresses one or more of the problems presented by prior art arrangements.

Remarkably, the invention provides a device which can be used with one or two part sanitising solutions. Advantageously, the invention provides a convenient package having premeasured amounts of reagents so that the correct amount of the required reagents is used.

### Summary of the Invention

In accordance with a first aspect of the present invention, there is provided a device comprising an enclosure, at least one applicator having a first liquid applied thereto and at least one vessel having a second liquid therein, the applicator and the vessel being provided in the enclosure wherein the vessel is impermeable to liquid and sealed prior to use and it is capable of being ruptured by applying force to it by manual pressure through the enclosure, thereby releasing the second liquid which reacts with the first liquid to provide a third liquid which comprises or consists of a sanitising agent wherein the first and second liquids are selected from sodium chlorite and citric acid; water and hypochlorous acid; and hydrogen peroxide and acetic acid; or wherein the third liquid is selected from chlorine dioxide, hypochlorous acid and peroxy-acetic acid.

The invention provides the advantage that the second liquid can be kept separate from the applicator until the time of use. For example, the applicator might have a small bacterial load that could inactivate a sanitising agent over a long period of time whereas the invention facilitates avoidance of inactivation.

Preferably, the enclosure is substantially impermeable to liquid. In addition, preferably the enclosure is sealed prior to use.

Preferably, the enclosure is in the form of a first bag, pouch or sachet. In this regard, the enclosure is preferably defined by two opposing surfaces of two walls. Preferably the edge of the walls are joined mechanically, by adhesive, or by welding. The walls can be of any shape. For example, in one embodiment, the walls are rectangular.

Preferably, the enclosure is substantially or fully flexible. The enclosure is preferably sufficiently robust to remain sealed if pressure is applied through the enclosure to open the vessel, for example by bursting the vessel.

Preferably the enclosure is at least partially or fully colourless or transparent. Alternatively, the enclosure is at least partially or fully opaque. In one preferred embodiment, the enclosure is partially colourless or transparent and partially opaque. This provides the advantage of allowing the applicator and the vessel to be seen through the wall of the enclosure. In one preferred embodiment, the surface of the enclosure is printed, by any commercial available method. For example, it is preferably printed with instructions for use of the device.

Preferably, the enclosure is of a polymeric material, more preferably of a polymeric film. Preferably, the polymeric film is selected from one or more of polyethylene, polypropylene PVC, co-extruded polyethylene/polyolefin LLDP, PET/PE peelable or non-peelable, PET/PE EVA or modified EVA, PET/Foil/PE peelable or non-peelable, polyamide/ LDPE peelable or non-peelable, coated paper/PE/Foil/Ionomer, LDPE 70 Int. film and OPA 15/LLDPE 70.

Preferably, the enclosure comprises a single layer or multiple layers produced by extrusion, co-extrusion, lamination, calendaring, casting or other commercial method. In one embodiment, the enclosure comprises multiple layers and they are of different polymers.

Preferably, the enclosure comprises a weakened strip or a tear notch or nick in the periphery of the enclosure. Preferably, the weakened strip, tear notch or nick is provided adjacent and parallel to one edge of the enclosure. This provides the advantage of easy opening of the enclosure. For example, the weakened strip can be provided by one or more of a line of at least partial perforations, a laser cut strip, a weakened seal. A weakened strip, tear notch or nick can provide the advantage of facilitating tearing open of the enclosure for removal of the applicator.

Preferably, the applicator is in the form of a sponge, foam pad, textile wipe, paper based towel, tissue, swab or swab stick. The applicator preferably comprises a handle, which is preferably of plastics material. The applicator is preferably natural or synthetic in origin or a combination thereof. More preferably, the applicator is a textile wipe. This provides the advantage of allowing liquid to be applied by the applicator to a surface, for example a hard surface, skin, or a wound.

Preferably, the applicator is folded. This provides the advantage of facilitating containment of the applicator within the enclosure.

Preferably, a single applicator is provided in the enclosure. Alternatively, a plurality of applicators are provided in the enclosure. Preferably, about 1 to about 500 applicators are provided in the enclosure. More preferably, about 1 to about 100 applicators are provided in the enclosure. Most preferably, about 1 to about 25 applicators are provided in the enclosure.

Preferably, the applicator or applicators is /are of a size sufficient to hold the liquid available contained in the enclosure after it has been released from the vessel for example by bursting the vessel. In addition, the applicator or applicators are sufficiently small to be wholly contained within the enclosure.

Preferably, the vessel is in the form of a second bag, pouch or sachet. In this regard, the vessel is preferably defined by two opposing surfaces of two walls. Preferably the edge of the walls are joined mechanically, by adhesive, or by welding. The walls can be of any shape. For example, in one embodiment, the walls are rectangular.

Preferably, the vessel is substantially or fully flexible. Alternatively, the vessel is substantially or fully rigid. Alternatively, the vessel is partially flexible and partially rigid. The vessel is preferably sufficiently robust to remain sealed until sufficient pressure is applied through the enclosure to open the vessel, for example by bursting the vessel or by breaking it into more than one piece.

Preferably the vessel is at least partially or fully colourless or transparent. Alternatively, the vessel is at least partially or fully opaque. In one preferred embodiment, the vessel is partially colourless or transparent and partially opaque. This provides the advantage of allowing the liquid contained by the vessel to be seen through the wall of the vessel. In one preferred embodiment, the surface of the vessel is printed, by a commercially available method.

Preferably, the vessel is of a polymeric material, more preferably of a polymeric film. Preferably, the polymeric film is selected from one or more of co-extruded polyethylene/polyolefin LLDP, PET/PE peelable or non-peelable, PET/PE EVA or modified EVA, PET/Foil/PE peelable or non-peelable, polyamide/ LDPE peelable or non-peelable, coated paper/PE/Foil/Ionomer, LDPE 70 Int. film and OPA 15/LLDPE 70.

Preferably, the vessel comprises a single layer or multiple layers produced by extrusion, co-extrusion, lamination, calendaring, casting or other commercial method. In one embodiment, the vessel comprises multiple layers and they are of different polymers.

Preferably, the vessel comprises a means to aid in opening or bursting of the vessel. The means comprises a weakened area or line in the wall of the vessel. Preferably, the means is provided at the edge of the vessel or adjacent and parallel to one edge of the vessel. This provides the advantage of easy opening of the vessel, for example by bursting the vessel and release of the liquid therein by applying pressure to the vessel through the enclosure. For example, the means can be provided by one or more of a line of at least partial perforations, a laser cut strip, or a weakened seal. For example, the weakened seal is provided by an interference strip which interferes with the ability to weld the seal. The means to aid in opening or bursting of the vessel ruptures preferentially on application of manual pressure to release liquid from within the vessel into the enclosure. Preferably, the vessel is loose within the enclosure. Alternatively, it is fixed to the inner wall of the enclosure. For example, it can be fixed to the wall of the enclosure by adhesive or by sharing a weld line with the enclosure. In one preferred embodiment, the vessel is fixed to the applicator. Alternatively, the vessel is not fixed to the applicator. Preferably, the vessel has an internal volume of about 1ml to about 500ml. More preferably it has an internal volume of about 1ml to about 300ml. Most preferably, it has an internal volume of about 1ml to about 100ml.

Preferably, two or more vessels are provided in the enclosure. The vessels may contain the same liquid or different liquids. The number of vessels provided is determined by the amount of liquid needed to provide sufficient liquid to wet the applicator. Alternatively, where according to the invention the vessel contains a first liquid and the applicator is pre-wetted with a second liquid, the number of vessels provided is determined by the amount of liquid required for reaction between the first and second liquids.

According to the present invention, the applicator is pre-wetted with a first liquid and the vessel contains a second liquid which reacts with the first liquid to provide a third liquid which comprises a sanitising agent. The third liquid may have different properties to the first or second liquids. For example, in an embodiment the third liquid is biocidal and / or fungicidal and / or has sporicidal properties. This provides the advantage of facilitating preparation of fresh sanitising agent shortly before or immediately before use. In addition, it enables a sanitising agent to be used that would otherwise render the materials of the vessel and / or the enclosure unsuitable for their purpose. For example the sanitising agent may attack or oxidise these materials so that on exposure to the sanitising agent they remain suitable for their purpose for only a short time.

In a further alternative embodiment, at least two vessels are provided and the vessels have different liquids therein. When the liquids are released from the vessels they react to provide the third liquid.

In an embodiment, the device of the invention is suitable for cleaning hard surfaces. For example, it can be used for cleaning a medical device.

In an alternative embodiment, the device of the invention is for veterinary or human disinfection and / or infection control. For example, it can be used as a biocidal, virucidal, sporicidal, fungicidal or a combination of two or more thereof.

### Brief Description of the Drawings

The invention will now be described in detail by reference to the accompanying drawings in which;

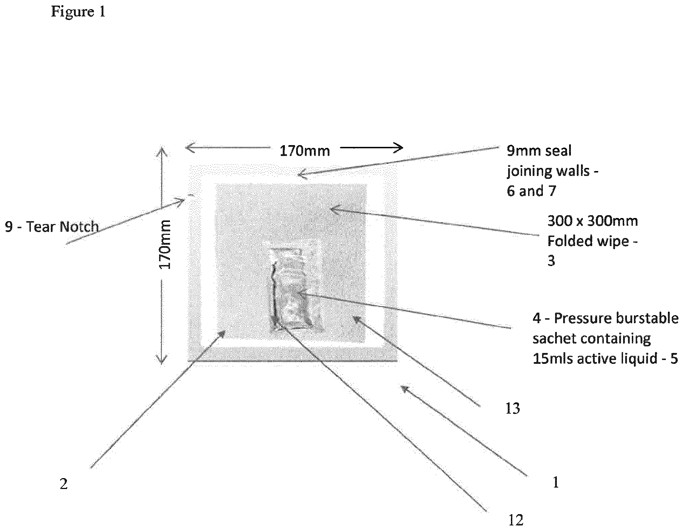

Figure 1 shows an example of a device according to the invention having a tear notch;

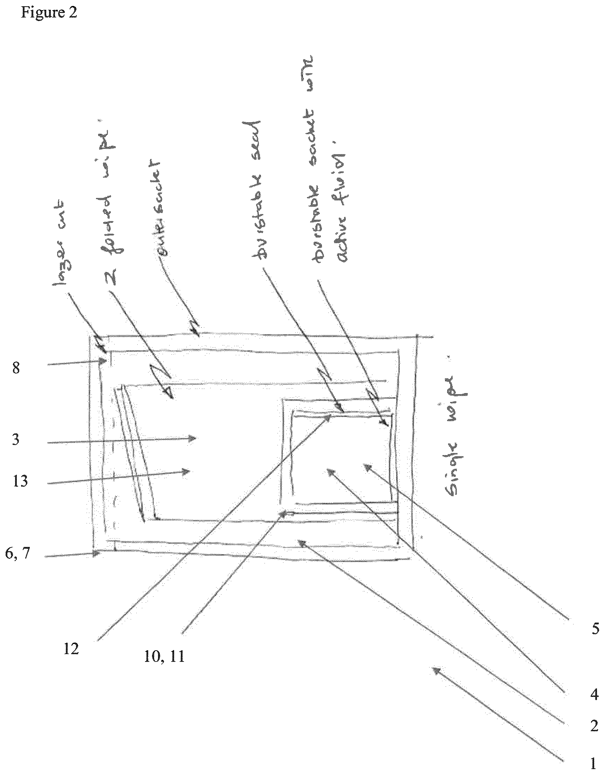

Figure 2 shows an example of a device according to the invention having a laser cut weakened strip and single applicator in the form of a wipe having two folds.

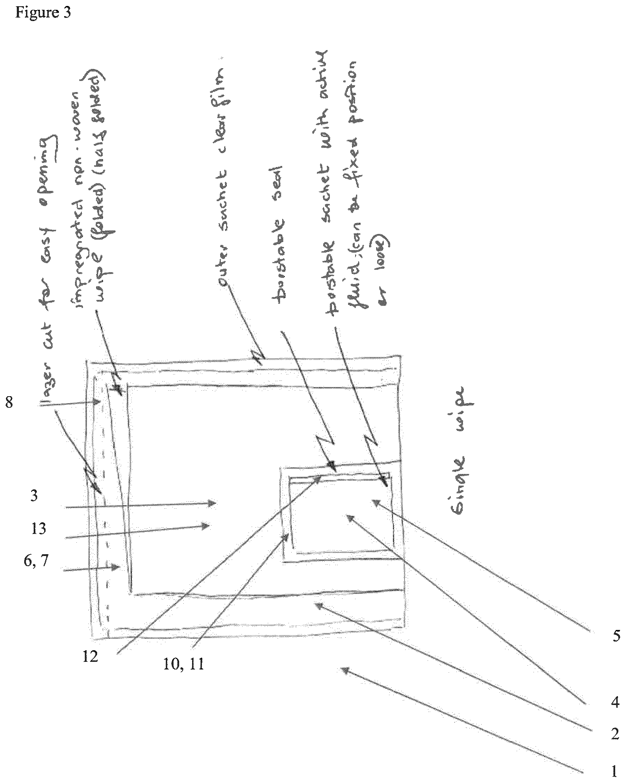

Figure 3 shows an example of a device according to the invention having a laser cut weakened strip and single applicator in the form of a wipe having a single fold pre-wetted with a liquid; and

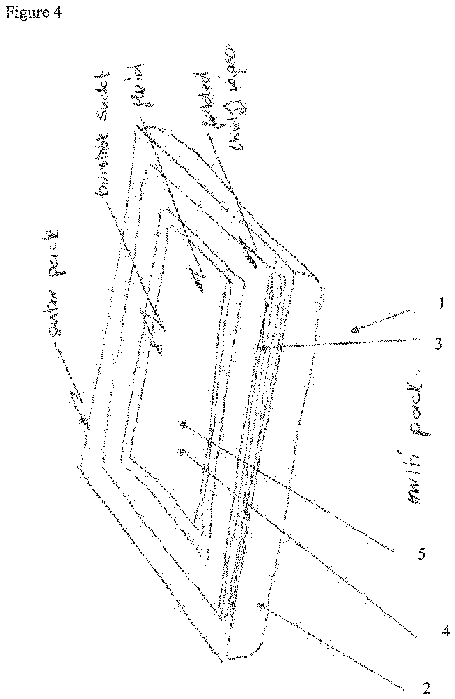

Figure 4 shows an example of a device according to the invention having a laser cut weakened strip and a plurality of applicators in the form of folded wipes.

### Detailed Description of the Invention

It will be appreciated that aspects, embodiments and preferred features of the invention have been described herein in a way that allows the specification to be written in a clear and concise way. However, unless circumstances clearly dictate otherwise, aspects, embodiments and preferred features can be variously combined or separated in accordance with the invention. In a preferred embodiment, a device in accordance with the invention comprises all aspects of the invention.

Within the context of this specification, the word "about" means preferably plus or minus 20%, more preferably plus or minus 10%, even more preferably plus or minus 5%, most preferably plus or minus 2%.

Within the context of this specification, the word "comprises" means "includes, among other things" and should not be construed to mean "consists of only".

Within the context of this specification, the word "substantially" means preferably at least 90%, more preferably 95%, even more preferably 98%, most preferably 99%.

Within the context of this specification, the term "an applicator having a liquid applied thereto", is taken to mean that a liquid is used to pre-wet an applicator. The applicator is saturated, partially saturated, soaked, partially soaked, moistened or wetted with the liquid. Alternatively, the liquid is absorbed or adsorbed onto the applicator.

Within the context of this specification, the term "sanitise" or "sanitising" refers to simultaneous cleaning and disinfection or antiseptic treatment. In addition, "disinfection" refers to the killing of microbials or microorganisms living on hard surfaces whereas "antiseptic treatment" refers to the killing of microbials or microorganisms on living tissue. Within the context of this specification, "water" is taken to mean substantially distilled or substantially purified water.

With reference to the figures, the invention provides a device (1) comprising an enclosure (2), at least one applicator (3) having a liquid (13) applied thereto and at least one vessel (4) having liquid (5) therein, wherein the applicator (3) and the vessel (4) are provided in the enclosure (2).

The enclosure (2) is in the form of a first sachet which is impermeable to liquid (5), (13) and sealed prior to use. In this regard, the enclosure (2) is defined by two opposing surfaces of two walls (6) and (7). The edges of the walls (6) and (7) are joined by welding. The walls (6) and (7) are rectangular.

The enclosure (2) is of a single layer of fully flexible polymeric film, but sufficiently robust to remain sealed if pressure is applied through the enclosure (2) to open the vessel (4), by bursting the vessel (4).

The enclosure (2) is partially colourless and partially opaque. In addition, the surface of the enclosure is printed with instructions for use of the device.

The enclosure (2) comprises a weakened strip (8) of a laser cut partial perforations or a tear notch (9) in the periphery of the enclosure. The weakened strip (8) or tear notch (9) is provided adjacent and parallel to one edge of the enclosure.

The applicator (3) is in the form of a folded textile wipe. In Example 1, described below, the applicator (3) is pre-wetted with a 13ml solution of stabilised hypochlorite (13) and in Example 2, the applicator (3) is pre-wetted with 13ml of purified water plus preservative benzalkonium chloride (13).

A single applicator (3) is provided or a plurality (for example about 1 to about 25) of applicators (3) are provided in the enclosure.

The applicator (3) or applicators (3) is /are square wipes when unfolded of about 300mm x 300mm, which has been found to be a size sufficient to hold the liquid available in the enclosure (2) after it has been released from the vessel (4) by bursting the vessel.

The vessel (4) is in the form of a second sachet which is impermeable to liquid (5) and sealed prior to use. In this regard, the vessel (4) is defined by two opposing surfaces of two walls (10) and (11). The edges of the walls (10) and (11) are joined by welding. The walls (10) and (11) are rectangular.

The vessel (4) is of a single layer of fully flexible polymeric film, but sufficiently robust to remain sealed until sufficient pressure is applied through the enclosure (2) to open the vessel (4), by bursting the vessel (4).

The vessel is partially colourless and partially opaque. In addition, the surface of the vessel is printed.

The vessel comprises a weakened strip in the wall of the vessel. In this regard, the seal (12) along one edge of the walls (10) and (11) is weakened so that it ruptures preferentially on application of manual pressure to release liquid (5) from the vessel (4) into the enclosure (2).

The vessel (4) is loose within the enclosure (2) and the vessel (2) is not fixed to the applicator (3).

The vessel has an internal volume of about 1ml to about 100ml.

The liquid (5) contained in the vessel (4) is released from the vessel (4) by rupturing the vessel (4) by applying force to the vessel (4) by manual pressure through the enclosure (2). This provides the advantage that the liquid (5) can be kept separate from the applicator (3) until the time of use. For example, the applicator might have a small bacterial load that could inactivate the sanitising agent over a long period of time whereas the invention facilitates avoidance of inactivation.

In one embodiment that is not shown in the figures, two or more vessels (4) are provided in the enclosure (2). The vessels (4) may contain the same liquid (5) or different liquids (5). The number of vessels (4) provided is determined by the amount of liquid (5) needed to provide sufficient liquid (5). Alternatively, where according to the invention, the applicator (3) is pre-wetted with a first liquid (13) and vessel (4) contains second liquid (5), the number of vessels (4) provided is determined by the amount of liquid (5) required for reaction between the first (13) and second liquids (5).

In Example 1, described below, the applicator (3) which is of HYN35 Polyprop/Viscose is pre-wetted with a first liquid (13) of a 13ml solution of stabilised hypochlorite and the vessel (4) contains a second liquid of a 13ml solution of citric acid (5) and which reacts with the first liquid (13) to provide a third liquid of chlorine dioxide which acts as a sanitising agent.

In Example 2, described below, the vessel (4) of Oriented Polyamide/LLDPE contains a liquid (5) of a 15ml solution of hypochlorous acid which acts as a sanitising agent.

In a further alternative embodiment which is not shown in the figures, at least two vessels (4) are provided and the vessels have different liquids (5) therein. When the liquids (5) are released from the vessels (4) they react to provide the third liquid.

The complete device may be presented non-sterile or it may be sterilised by a suitable method where required. For example, the device can be sterilised by a method selected from gamma irradiation, X-Ray irradiation, steam and electron beam treatment.

### EXAMPLES

### Example 1

In a first example wherein the active sanitising reagent is chlorine dioxide, the following materials were used to provide a device according to the invention:

| | |
|---|---|
| Enclosure (outer sachet): - | PET / PE (polyester / polyethylene) |
| Applicator (wipe): - | HYN35 Polyprop/Viscose |
| Vessel (inner sachet): - | Oriented Polyamide/LLDPE |
| Interference strip: - | 70gm LLDPE |
| First liquid (inner sachet dose): - | Citric Acid (13mls) |
| Second liquid (impregnated into wipe): - | Stabilised Hypochlorite (13mls) |

### Example 2

In a second example wherein the active sanitising reagent is hypochlorous acid, the following materials were used to provide a device according to the invention:

| | |
|---|---|
| Enclosure (outer sachet): - | PET / PE (polyester / polyethylene) |
| Applicator (wipe): - | HYN35 Polyprop/Viscose |
| Vessel (inner sachet): - | Oriented Polyamide/LLDPE |
| Interference strip: - | 70gm LLDPE |
| First liquid (inner sachet dose): - | Hypochlorous Acid (15mls) |
| Second liquid (impregnated into wipe): - | Purified Water plus preservative Benzalkonium Chloride (13mls) |

### Test Data

### Test 1: Investigation into the Volume of Liquid Required

### Testing Details

In order to produce the most readily visible results, it was decided that a 5% aqueous solution of Potassium Permanganate (KMnO₄) would be used as the liquid on test. This liquid was selected because it gives a vivid purple colour when wet, easily showing the extent of liquid uptake, but also because it leaves brown staining when dry, allowing samples to be stored and analysed in the future.

In all cases, testing was conducted by measuring the specified volume of liquid in a measuring cylinder and pouring the liquid centrally onto a quarter folded wipe. The wipes were left, unagitated, to soak up the liquid in the bottom of a plastics container for 20 seconds before removal, upon which they were photographed and dried.

### Test Results

The initial testing consisted of pouring specified volumes of liquid on a dry wipe until it was saturated. From this testing it was determined that 26ml of liquid was required to fully wet a wipe within a 20 second timeframe.

Following this, for comparison purposes, a wipe was soaked with 13ml of liquid. The resultant wipe showed a soaked area of around half the wipe. This showed that the uptake of liquid by the wipe was approximately linear.

In the next test, the potential use of impregnated wipes was investigated by pre-soaking a wipe with water and then applying further water. Water having 50% of the total volume of permanganate solution determined above was used for pre-soaking the wipe and water having a further 50% of the total volume was applied thereafter. For example, for a 26ml total volume, the wipe was wetted with 13ml water before 13ml additional water was applied. To investigate whether a pre-soak had an effect on total liquid uptake by a wipe, it was decided that a reduced total volume, of 22ml, would be tested initially.

It was seen that the pre-soak did not compensate for the reduced volume of the total liquid, and resulted in production of a wipe having dry patches.

The effect of 26ml total volume liquid was tested, allowing a comparison to be drawn between the 26ml applied to a dry wipe without pre-wetting. It was found that application of 13ml of water to a wipe pre-wetted with 13ml water produced a less effective distribution compared to a single application of 26ml solution.

Finally a total volume of 30ml water was tested. It was seen that the dispersion of liquid was not even throughout the wipe, as indicated by lighter patches in the top left hand corner of the wipe, and the liquid did not fully wet the wipe.

To conclusively prove that an application of 15ml solution to a wipe pre-wetted with 15 ml solution would fully cover the wipe, it was decided that the 15ml of water used to pre-wet the wipe should be changed for potassium permanganate.

The results of this test, produced a very dark, almost black, purple colouration, and this showed that application of 15ml potassium permanganate solution to pre-wet the wipe followed by 15ml water was sufficient to fully soak the entire wipe.

Running in parallel with the above tests, an investigation into the volume to wet different wipe materials was conducted. Wipes of Sontara and Polycotton were tested. In addition, these tests were conducted based on a mass of liquid required rather than volume and visual basis. From these results assuming the total volume required to wet the wipe would be split into half so that 50% of the volume would be used to pre-wet the wipe and 50% would be applied thereafter by bursting the vessel, the minimum volume of liquid required in the vessel to wet both wipes was determined to be 12.67ml, and this was rounded to 13ml. This corresponds favourably to the 15ml value determined above.

| | | Dry | Wet | | |
|---|---|---|---|---|---|
| | | Mass | Mass | Vol. of Wetout | Therefore Vol. of Sachet |
| Sontara | 1 | 5.3 | 30.55 | 25.25 | 12.625 |
| | 2 | 5.47 | 30.59 | 25.12 | 12.56 |
| | 3 | 5.52 | 31.18 | 25.66 | 12.83 |
| | Avg | 5.43 | 30.77 | 25.34 | 12.67 |

| | | | | | |
|---|---|---|---|---|---|
| Polycotton | 1 | 3.39 | 20.04 | 16.65 | 8.325 |
| | 2 | 3.42 | 21.09 | 17.67 | 8.835 |
| | 3 | 3.46 | 21.68 | 18.22 | 9.11 |
| | Avg | 3.42 | 20.94 | 17.51 | 8.76 |

### Conclusion and Discussion

Based on the idealised environment of laboratory testing it was found that the minimum volume of liquid required to fully wet a standard, quarter folded, wipe was 26ml, with 30ml being preferred for an impregnated wipe. Assuming the total volume required to wet the wipe would be split into half so that 50% of the volume would be used to pre-wet the wipe and 50% would be applied thereafter by bursting the vessel, this corresponds to vessel liquid volumes of 13ml and 15ml respectively.

### Tensile Testing

Tensile testing using a Mecmesin Tensile Test has revealed that the peel strength for opening the enclosure should be:
From about 0.5 Kgf to about 1.2 Kgf

Tensile testing using a Mecmesin AFG Force Gauge has revealed that the force required to burst the vessel should be:
From about 25 Kgf to about 35 Kgf.

## Claims

1. A device comprising an enclosure, at least one applicator having a first liquid applied thereto and at least one vessel having a second liquid therein, the applicator and the vessel being provided in the enclosure wherein the vessel is impermeable to liquid and sealed prior to use and it is capable of being ruptured by applying force to it by manual pressure through the enclosure, thereby releasing the second liquid which reacts with the first liquid to provide a third liquid which comprises or consists of a sanitising agent wherein the first and second liquids are selected from sodium chlorite and citric acid; water and hypochlorous acid; and hydrogen peroxide and acetic acid; or wherein the third liquid is selected from chlorine dioxide, hypochlorous acid and peroxy-acetic acid.

2. The device according to claim 1, wherein the enclosure is substantially impermeable to liquid.

3. The device according to any one of the preceding claims, wherein the enclosure is sealed prior to use

4. The device according to any one of the preceding claims, wherein the enclosure is in the form of a first bag, pouch or sachet.

5. The device according to any one of the preceding claims, wherein the enclosure is defined by two opposing surfaces of two walls.

6. The device according to any one of the preceding claims, wherein the enclosure is fully flexible.

7. The device according to any one of the preceding claims, wherein the enclosure is at least partially or fully colourless or transparent; or partially colourless or transparent and partially opaque.

8. The device according to any one of the preceding claims, wherein the enclosure is of a polymeric material.

9. The device according to claim 8, wherein the polymeric material is selected from one or more of polyethylene, polypropylene, PVC, co-extruded polyethylene/polyolefin LLDP, PET/PE peelable or non-peelable, PET/PE EVA or modified EVA, PET/Foil/PE peelable or non-peelable, polyamide/ LDPE peelable or non-peelable, coated paper/PE/Foil/Ionomer, LDPE 70 Int. film and OPA 15/LLDPE 70.

10. The device according to any one of the preceding claims wherein the enclosure comprises a single layer or multiple layers.

11. The device according to any one of the preceding claims, wherein the enclosure comprises a weakened strip or a tear notch or nick in the periphery of the enclosure.

12. The device according to any one of the preceding claims wherein the applicator is in the form of a sponge, foam pad, textile wipe, paper based towel, tissue, swab or swab stick.

13. The device according to any one of the preceding claims wherein a single applicator is provided in the enclosure or a plurality of applicators are provided in the enclosure.

14. The device according to any one of the preceding claims, wherein, prior to use, the vessel is in the form of a second bag, pouch or sachet.

15. The device according to any one of the preceding claims, wherein, prior to use, the vessel is defined by two opposing surfaces of two walls.

16. The device according to any one of the preceding claims, wherein, prior to use, the vessel is flexible; rigid; or partially flexible and partially rigid.

17. The device according to any one of the preceding claims, wherein, prior to use, the vessel is at least partially or fully colourless or transparent; or partially colourless or transparent and partially opaque.

18. The device according to any one of the preceding claims, wherein, prior to use, the vessel is of a polymeric material.

19. The device of claim 18, wherein the polymeric material of the vessel is selected from one or more of co-extruded polyethylene/polyolefin LLDP, PET/PE peelable or non-peelable, PET/PE EVA or modified EVA, PET/Foil/PE peelable or non-peelable, polyamide/ LDPE peelable or non-peelable, coated paper/PE/Foil/Ionomer, LDPE 70 Int. film and OPA 15/LLDPE 70.

20. The device according to any one of the preceding claims wherein the vessel comprises a means to aid in opening or bursting of the vessel.

21. The device of claim 20, wherein means to aid in opening or bursting of the vessel comprises a weakened area or line in the wall of the vessel.

22. The device of claim 21, wherein the means is provided by one or more of a line of at least partial perforations, a laser cut strip, or a weakened seal.

23. The device according to any one of the preceding claims wherein the vessel is loose within the enclosure; fixed to the inner wall of the enclosure or fixed to the applicator.

24. The device according to any one of the preceding claims wherein the vessel has an internal volume of 1ml to 500ml.

25. The device according to any one of the preceding claims wherein two or more vessels are provided in the enclosure.

26. The device according to claim 25, wherein the vessels may contain the same liquid or different liquids.

27. The device according to any one of the preceding claims for use in cleaning hard surfaces or for use in veterinary or human disinfection and / or infection control.

## Patentansprüche

1. Gerät, welches umfasst, ein Gehäuse, mindestens einen Applikator mit einer darauf aufgebrachten ersten Flüssigkeit und mindestens einen Behälter mit einer zweiten Flüssigkeit darin, worin der Applikator und der Behälter in dem Gehäuse vorgesehen sind,
worin der Behälter für Flüssigkeit undurchlässig und vor Verwendung versiegelt ist und durch Ausüben von Kraft darauf mittels manuellen Drucks durch den Behälter aufgerissen werden kann, wodurch die zweite Flüssigkeit freigesetzt wird, die mit der ersten Flüssigkeit reagiert, um eine dritte Flüssigkeit bereitzustellen, die ein Desinfektionsmittel umfasst oder daraus besteht,
worin die erste und zweite Flüssigkeit ausgewählt ist unter Natriumchlorit und Zitronensäure; Wasser und hypochloriger Säure; und Wasserstoffperoxid und Essigsäure; oder
worin die dritte Flüssigkeit ausgewählt ist unter Chlordioxid, hypochloriger Säure und Peroxyessigsäure.

2. Gerät nach Anspruch 1, worin der Behälter für Flüssigkeiten im Wesentlichen undurchlässig ist.

3. Gerät nach einem der vorstehenden Ansprüche, worin der Behälter vor Verwendung versiegelt ist.

4. Gerät nach einem der vorstehenden Ansprüche, worin der Behälter in Form eines ersten Sacks, einer Tasche oder eines Beutels ist.

5. Gerät nach einem der vorstehenden Ansprüche, worin der Behälter durch zwei gegenüberliegende Oberflächen von zwei Wänden definiert ist.

6. Gerät nach einem der vorstehenden Ansprüche, worin der Behälter vollständig flexibel ist.

7. Gerät nach einem der vorstehenden Ansprüche, worin der Behälter mindestens teilweise oder vollständig farblos oder transparent ist; oder teilweise farblos oder transparent und teilweise durchsichtig ist.

8. Gerät nach einem der vorstehenden Ansprüche, worin der Behälter aus einem Polymermaterial besteht.

9. Gerät nach Anspruch 8, worin das Polymermaterial ausgewählt ist unter einem oder mehreren von Polyethylen, Polypropylen, PVC, ko-extrudiertem Polyethylen/Polyolefin LLDP, abziehbarem oder nicht-abziehbarem PET/PE, PET/PE EVA oder modifiziertem EVA, abziehbarem oder nicht-abziehbarem PET/Folie/PE, Polyamid/ abziehbarem oder nicht-abziehbarem LDPE, beschichtetem Papier/PE/Folie/Ionomer, LDPE 70 Int. Film und OPA 15/LLDPE 70.

10. Gerät nach einem der vorstehenden Ansprüche worin der Behälter eine einzelne Schicht oder mehrere Schichten umfasst.

11. Gerät nach einem der vorstehenden Ansprüche, worin der Behälter einen Sollbruch-Streifen oder eine Einreißkerbe oder -Einbuchtung im Umfang des Behälters umfasst.

12. Gerät nach einem der vorstehenden Ansprüche worin der Applikator in der Form eines Schwamms, eines Schwamm-Pads, eines Gewebe-Tuchs, eines Papiertuchs, Gewebe, Tupfer oder Tupferstabs vorliegt.

13. Gerät nach einem der vorstehenden Ansprüche worin in dem Behälter ein einzelner Applikator vorgesehen ist oder in dem Behälter mehrere Applikatoren vorgesehen sind.

14. Gerät nach einem der vorstehenden Ansprüche, worin der Behälter vor Verwendung in der Form eines zweiten Sacks, Tasche oder Beutels ist.

15. Gerät nach einem der vorstehenden Ansprüche, worin vor Verwendung der Behälter durch zwei gegenüberliegende Oberflächen von zwei Wänden definiert ist.

16. Gerät nach einem der vorstehenden Ansprüche, worin der Behälter vor Verwendung flexibel, fest oder teilweise flexibel und teilweise fest ist.

17. Gerät nach einem der vorstehenden Ansprüche, worin der Behälter vor Verwendung mindestens teilweise oder vollständig farblos oder transparent ist; oder teilweise farblos oder transparent und teilweise durchsichtig ist.

18. Gerät nach einem der vorstehenden Ansprüche, worin der Behälter vor Verwendung aus einem Polymermaterial besteht.

19. Gerät nach Anspruch 18, worin das Polymermaterial des Behälters ausgewählt ist unter einen oder mehreren von ko-extrudiertem Polyethylen/Polyolefin LLDP, abziehbarem oder nicht-abziehbarem PET/PE, PET/PE EVA oder modifiziertem EVA, abziehbarem oder nicht-abziehbarem PET/Folie/PE, abziehbarem oder nicht-abziehbarem Polyamid/LDPE, beschichtetem Papier/PE/Folie/Ionomer, LDPE 70 Int. Film und OPA 15/LLDPE 70.

20. Gerät nach einem der vorstehenden Ansprüche worin der Behälter Mittel zur Unterstützung des Öffnens oder Zerbrechens des Behälters umfassen.

21. Gerät nach Anspruch 20, worin die Mittel zur Unterstützung des Öffnens oder Zerbrechens des Behälter einen geschwächten Bereich oder eine geschwächte Linie in der Wand des Behälters umfassen.

22. Gerät nach Anspruch 21, worin das Mittel durch ein oder mehrere von einer Linie von mindestens einer teilweisen Perforation, einem mit Laser-geschnittenen Streifen, oder einem geschwächten Siegel bereitgestellt wird.

23. Gerät nach einem der vorstehenden Ansprüche, worin der Behälter lose in dem Behälter, an der Innenwand des Behälters befestigt oder am Applikator befestigt ist.

24. Gerät nach einem der vorstehenden Ansprüche worin der Behälter ein Innenvolumen von 1ml bis 500ml aufweist.

25. Gerät nach einem der vorstehenden Ansprüche worin zwei oder mehrere Behälter in dem Behälter vorgesehen sind.

26. Gerät nach Anspruch 25, worin die Behälter die gleiche Flüssigkeit oder unterschiedliche Flüssigkeiten enthalten können.

27. Gerät nach einem der vorstehenden Ansprüche zur Verwendung bei der Reinigung von harten Oberflächen oder zur Verwendung bei der Tier- oder Human-Desinfektion und/oder Infektionskontrolle.

## Revendications

1. Dispositif comprenant une enceinte, au moins un applicateur ayant un premier liquide appliqué sur celui-ci et au moins un récipient ayant un deuxième liquide dans celui-ci, l'applicateur et le récipient étant disposés dans l'enceinte,
dans lequel le récipient est imperméable aux liquides et scellé de façon étanche avant utilisation et est capable d'être rompu par application d'une force à celui-ci par pression manuelle à travers l'enceinte, permettant ainsi de libérer le deuxième liquide qui réagit avec le premier liquide pour fournir un troisième liquide qui comprend ou consiste en un agent de désinfection,
dans lequel les premier et deuxième liquides sont choisis parmi le chlorite de sodium et l'acide citrique ; l'eau et l'acide hypochloreux ; et le peroxyde d'hydrogène et l'acide acétique ; ou
dans lequel le troisième liquide est choisi parmi le dioxyde de chlore, l'acide l'hypochloreux et l'acide peroxy - acétique.

2. Dispositif selon la revendication 1, dans lequel l'enceinte est sensiblement imperméable aux liquides.

3. Dispositif selon l'une quelconque des revendications précédentes, dans lequel l'enceinte est scellée de façon étanche avant utilisation.

4. Dispositif selon l'une quelconque des revendications précédentes, dans lequel enceinte est dans la forme d'un premier sac, poche ou sachet.

5. Dispositif selon l'une quelconque des revendications précédentes, dans lequel l'enceinte est définie par deux surfaces opposées de deux parois.

6. Dispositif selon l'une quelconque des revendications précédentes, dans lequel l'enceinte est totalement flexible.

7. Dispositif selon l'une quelconque des revendications précédentes, dans lequel l'enceinte est au moins partiellement ou totalement incolore ou transparente ; ou partiellement incolore ou transparente et partiellement opaque.

8. Dispositif selon l'une quelconque des revendications précédentes, dans lequel l'enceinte est d'une matière polymère.

9. Dispositif selon la revendication 8, dans lequel la matière polymère est choisie parmi un ou plusieurs parmi le polyéthylène, le polypropylène, le PVC, le polyéthylène/polyoléfine LLDP coextrudé, le PET/PE pelable ou non pelable, le PET/PE EVA ou EVA modifié, le PET/Feuille de métal/PE pelable ou non pelable, le polyamide/LDPE pelable ou non pelable, le papier couché/PE/Feuille de métal/Ionomère, le LDPE 70 Int. film et l'OPA 15/LLDPE 70.

10. Dispositif selon l'une quelconque des revendications précédentes, dans lequel l'enceinte comprend une simple couche ou de multiples couches.

11. Dispositif selon l'une quelconque des revendications précédentes, dans lequel l'enceinte comprend une bande affaiblie ou une encoche ou coupure de déchirure dans la périphérie de l'enceinte.

12. Dispositif selon l'une quelconque des revendications précédentes, dans lequel l'applicateur est dans la forme d'une éponge, d'un tampon en mousse, d'une lingette textile, d'une serviette à base de papier, d'un papier-mouchoir, d'un écouvillon ou d'un bâtonnet de prélèvement.

13. Dispositif selon l'une quelconque des revendications précédentes, dans lequel un seul applicateur est disposé dans l'enceinte ou plusieurs applicateurs sont disposés dans l'enceinte.

14. Dispositif selon l'une quelconque des revendications précédentes, dans lequel, avant utilisation, le récipient est dans la forme d'un second sac, poche ou sachet.

15. Dispositif selon l'une quelconque des revendications précédentes, dans lequel, avant utilisation, le récipient est défini par deux surfaces opposées de deux parois.

16. Dispositif selon l'une quelconque des revendications précédentes, dans lequel, avant utilisation, le récipient est flexible ; rigide ; ou partiellement flexible et partiellement rigide.

17. Dispositif selon l'une quelconque des revendications précédentes, dans lequel, avant l'utilisation, le récipient est au moins partiellement ou totalement incolore ou transparent ; ou partiellement incolore ou transparent et partiellement opaque.

18. Dispositif selon l'une quelconque des revendications précédentes, dans lequel, avant utilisation, le récipient est d'une matière polymère.

19. Dispositif selon la revendication 18, dans lequel la matière polymère du récipient est choisie parmi un ou plusieurs parmi le polyéthylène/polyoléfine LLDP coextrudé, le PET/PE pelable ou non pelable, le PET/PE EVA ou EVA modifié, le PET/Feuille de métal/PE pelable ou non pelable, le polyamide/LDPE pelable ou non pelable, le papier couché/PE/Feuille de métal/Ionomère, le LDPE 70 Int. film et l'OPA 15/LLDPE 70.

20. Dispositif selon l'une quelconque des revendications précédentes, dans lequel le récipient comprend un moyen pour aider à ouvrir ou rompre le récipient.

21. Dispositif selon la revendication 20, dans lequel le moyen pour aider à ouvrir ou rompre le récipient comprend une zone ou ligne affaiblie dans la paroi du récipient.

22. Dispositif selon la revendication 21, dans lequel le moyen est fourni par un ou plusieurs parmi une ligne de perforations au moins partielles, une bande découpée au laser ou un joint affaibli.

23. Dispositif selon l'une quelconque des revendications précédentes, dans lequel le récipient est lâche à l'intérieur de l'enceinte ; fixé à la paroi interne de l'enceinte ou fixé à l'applicateur.

24. Dispositif selon l'une quelconque des revendications précédentes, dans lequel récipient a un volume interne de 1 ml à 500 ml.

25. Dispositif selon l'une quelconque des revendications précédentes, dans lequel au moins deux récipients sont disposés dans l'enceinte.

26. Dispositif selon la revendication 25, dans lequel les récipients peuvent contenir le même liquide ou des liquides différents.

27. Dispositif selon l'une quelconque des revendications précédentes pour une utilisation dans le nettoyage de surfaces dures ou pour une utilisation en désinfection vétérinaire ou humaine et / ou contrôle des infections vétérinaires ou humaines.
